(19) Europäisches Patentamt
European Patent Office
Office européen des brevets

(11) EP 0 726 255 A2

(12) EUROPEAN PATENT APPLICATION

(43) Date of publication:
14.08.1996 Bulletin 1996/33

(21) Application number: 96104311.4

(22) Date of filing: 14.05.1993

(51) Int. Cl.$^6$: C07D 237/18, C08K 5/37,
C08K 5/378, C08K 5/3462,
C08K 5/44, C08L 21/00

(84) Designated Contracting States:
AT BE CH DE DK ES FR GB GR IE IT LI LU MC NL
PT SE

(30) Priority: 05.06.1992 US 894677
04.05.1993 US 53971

(62) Application number of the earlier application in
accordance with Art. 76 EPC: 93911338.7

(71) Applicant: MONSANTO COMPANY
St. Louis Missouri 63167 (US)

(72) Inventors:
• Lin, Horng-Jau
  Wadsworth, Ohio 44281 (US)

• Rostek, Charles John, Jr.
  Bentleyville, Ohio 44022 (US)
• Sikora, David John
  Hudson, Ohio 44236 (US)

(74) Representative: Bosch, Henry et al
Monsanto Services International S.A.
Patent Department
Avenue de Tervuren 270/272
Letter Box No. 21
1150 Brussels (BE)

Remarks:
This application was filed on 19 - 03 - 1996 as a
divisional application to the application mentioned
under INID code 62.

(54) 3-Pyridazine derivatives and their use in rubber

(57) Thiols, disulfides and sulfenamides of certain
pyridazines are effective accelerators in the sulfur vul-
canization of rubber. These compounds, based on 3-
pyridazine show improved rates of vulcanization and
states of cure compared with known sulfenamide accel-
erators.

VULCANIZATION PARAMETERS

## Description

FIELD OF THE INVENTION

This invention relates to certain 3-pyridazine thiols, disulfides and sulfenamides and to their use in rubber.

BACKGROUND

A number of heterocyclic sulfenamides, thiols and disulfides have been well known, as has been their use in the vulcanization of rubber. The best known and most widely used are based on benzothiazole. Thus, the benzothiazole sulfenamides, such as N-t-butyl-2-benzothiazole sulfenamide (TBBS) and N-cyclohexyl-2-benzothiazole sulfenamide (CBS) have become standard accelerators of vulcanization. Similarly, the thiol derivative, 2-mercaptobenzothiazole (MBT) and the disulfide derivative, 2,2'-benzothiazole disulfide (MBTS), are standards of the industry.

To a lesser degree, other N-heterocycles have been suggested as the basis for sulfenamides. For example, British Patent 795,174 describes a process for making a large variety of sulfonamide compounds for use as diuretics and antibacterial agents in which the sulfenamide equivalent is first made as an intermediate. Twenty-six different basic heterocycles are suggested, and a wide variety of substituents and fused ring variations are included, as well.

Similarly, British Patent 1,342,046 discloses a process for making heterocyclic sulfenamides, based on diazine, triazine and pyridine thiols, encompassing an unlimited number of possible compounds, suggested to be effective vulcanization accelerators for rubber.

SUMMARY OF THE INVENTION

It has now been found that thiols, disulfides and sulfenamides based on certain 3-pyridazyl moieties are particularly effective accelerators for the vulcanization of natural and synthetic rubber. More particularly, 3-pyridazine thiols, disulfides and sulfenamides have been found to have superior accelerating effect on the vulcanization of natural and synthetic rubber, compared with similar compounds based on other heterocycles.

The compounds of the present invention when utilized as accelerators for curing natural rubber, synthetic rubbers such as polybutadiene, EPDM or styrene-butadiene rubber, blends of rubbers such as natural rubber and polybutadiene, styrene-butadiene rubber and polybutadiene, or combinations thereof, result in improved cure rates as indicated by t90-t2 values, t25-t2 values and maximum rate of vulcanization (Vmax), and higher extent of cure (cure efficiency), in comparison with traditional or conventional sulfenamide accelerators. Increased cure rates are very desirable since faster rates of production of rubber articles can be obtained. Molded rubber articles, such as tires, can thus be removed from the mold at earlier times without the risk of undercure. Higher extents of cure may negate the use of sulfur donors.

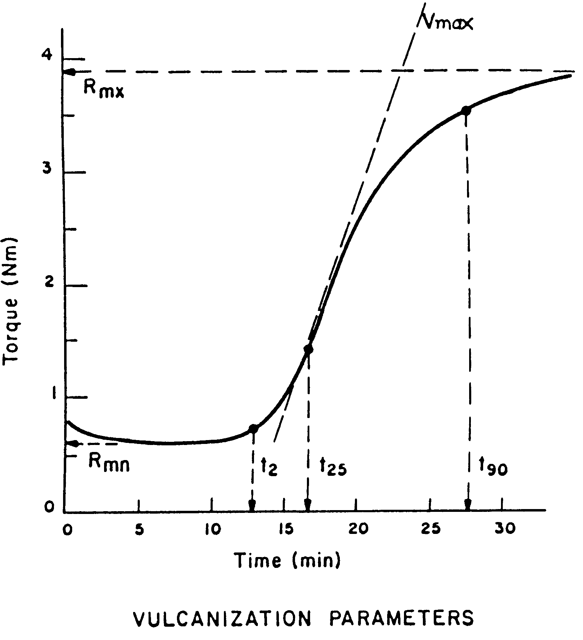

BRIEF DESCRIPTION OF THE DRAWING

The drawing is a typical rheograph showing the parameters of the vulcanization reaction.

DETAILED DESCRIPTION OF THE INVENTION

The compounds of the invention, which are used in rubber compositions to produce improved vulcanization behavior and/or improved vulcanizate properties, are based on 3-pyridazine (3-(1,2-diazine)).

A general formula for the compounds of the invention is

$$(PdS)_x(NRR')_yR''_z$$

wherein Pd is 3-pyridazyl, optionally substituted on the nucleus by one or more halogen atoms or lower alkoxy or hydroxyl groups; R is H or $C_{1-8}$ alkyl, $C_{3-8}$ cycloalkyl, phenyl, $C_{7-12}$ aralkyl or $C_{7-12}$ alkaryl; R' is H or R, or R and R' together with N form a heterocyclic ring; x can be 1 or 2, y can be 0 or 1 and z can be 0 or 1; provided that when x is 2, both y and z are 0, when x is 1 and y is 1, z is 0; and when x is 1 and y is 0, z is 1 and R'' is H or a cation, further provided that when z is 1, Pd contains at least one of the substituents recited above. This general formula encompasses the thiols and disulfides as well as the sulfenamides of the invention.

The thiol versions of these compounds have an -SH group attached at the 3 position, and are 3-pyridazine thiols containing one or more of the recited substituents on the nucleus. It is understood that the incorporation into the compositions of the invention of these substituted thiols can include their use in salt form; that is, as metal salts of these thiols (e.g., zinc thiolate salts) or quaternary ammonium salts of these thiols.

Similarly, the disulfides of the invention are 3,3'-pyridazine disulfides optionally containing one or more of the recited substituents on the nucleus.

The sulfenamide compounds of the invention all have a sulfenamide group attached at the 3 position, such that this sulfenamide group is based on a primary or secondary amine. The primary amines which can be used include $C_{1-8}$ alkyl amines such as methylamine, ethylamine, n-propylamine, isopropylamine, n-butylamine, sec-butylamine, isobutylamine, t-butylamine, n-amylamine, t-octylamine and the like; $C_{3-8}$ cycloalkylamines such as cyclopropylamine, cyclohexylamine, cyclooctylamine and the like; phenylamine (aniline); $C_{7-12}$ aralkylamines such as benzylamine and the like; and $C_{7-12}$ alkarylamines such as p-t-butylaniline and the like. Secondary amines include diisopropylamine, dicyclohexylamine and the like.

As stated above one or more non-reactive substituents can be present at the open positions on the 3-pyridazine ring, such as halogen atoms, lower alkoxy or hydroxyl groups. In the thiols of the invention, at least one of such substituents is necessarily present on the nucleus, while they are optional in the sulfenamides and disulfides.

Preferred sulfenamides of the invention are those made from isopropylamine, t-butylamine or cyclohexylamine; and thus include N-isopropyl-3-pyridazine sulfenamide, N-t-butyl-3-pyridazine sulfenamide, N-cyclohexyl-3-pyridazine sulfenamide, and the like.

The disulfides of the invention, namely,3,3'-dipyridazine disulfides, with and without the halogen, alkoxy and hydroxy substituents, can be prepared from their respective thiols by oxidation.

The sulfenamides of the invention can be prepared from the respective thiol or disulfide by treatment with amine in the presence of silver nitrate or an oxidizing agent such as sodium hypochlorite or oxygen.

The 3-pyridazine compounds of the present invention can be used as primary or auxiliary accelerators in the vulcanization of rubber. Generally any type of sulfur vulcanizable rubber can be utilized such as natural rubber, synthetic rubber, various blends of synthetic rubber and combinations thereof. Natural rubber is usually obtained from hevea Brasiliensis trees, and is generally grown in the tropics. Synthetic rubbers include those made from various dienes such as those having from 4 to 12 carbon atoms and preferably from 4 to 8 carbon atoms including 1,3-butadiene, isoprene, 2,3-dimethyl-1,3-butadiene, 2-methyl-1,3-pentadiene, 3,4-dimethyl-1,3-hexadiene, 4,5-diethyl-1,3-octadiene, phenyl-1,3-butadiene, pentadiene, hexadiene, octadiene, and the like. Synthetic rubbers also include copolymers made from the immediately above-noted dienes having from 4 to 12 carbon atoms with a vinyl substituted aromatic compound having from 8 to 20 carbon atoms such as styrene, alpha-methylstyrene, 4-n-propylstyrene, 4-t-butylstyrene, and the like, as well as copolymers made from the above dienes and acrylonitrile.

Another class of synthetic rubbers which can be utilized in the present invention are EPDM rubbers. These are polymers made from ethylene, propylene, and a minor proportion of a non-conjugated diene monomer such as ethylidenenorbornene, dicyclopentadiene, 1,4-hexadiene and the like. Butyl rubbers, which are copolymers from isobutylene and a minor proportion of isoprene, can be used, as well as their halogenated derivatives, such as chlorobutyl or bromobutyl rubber. Other sulfur vulcanizable rubbers known to the art and to the literature can also be utilized.

The rubber polymers made from conjugated dienes or copolymers of a conjugated diene or the vinyl substituted aromatic are preferably "elastomeric" materials, that is they conform, when vulcanized, to the definition of an elastomeric or rubber material found in ASTM D 1566.

As noted above, either natural rubber, one or more synthetic rubbers, that is either a single type of synthetic rubber or blends of two or more synthetic rubbers, as well as a blend of natural rubber and one or more synthetic rubbers can be cured utilizing one of the 3-pyridazine compounds of the present invention as a primary accelerator. When utilized as a primary accelerator, the amount thereof is generally from about 0.1 to about 10 parts and preferably from about 0.2 to about 2.0 parts by weight per 100 parts by weight (phr) of the rubber polymer or blend. When the 3-pyridazine compounds of the invention are utilized as accelerators for curing rubber compounds, the natural or synthetic rubber compositions of the present invention generally contain other conventional compounding ingredients in conventional amounts, both of which are well known to the art and to the literature. Sulfur, and/or a sulfur-donor in amounts of from 0.2 to 5 phr, is usually employed. Also, various fillers and reinforcing agents, such as clay, silica, and carbon black, can be utilized in amounts from 5 up to about 200 phr. Various oils, for example aromatic, naphthenic, or paraffinic, can be utilized to plasticize the rubber in amounts from 5 up to about 200 phr. Various activators such as zinc oxide, stearic acid, and the like, can also be used in amounts up to about 15 or more phr. Various antidegradants, and the like, well known in the art, can also be utilized. Such materials are generally mixed into the rubber by utilizing a mill, a Banbury mixer, or the like.

The rubber compositions can be used in a large number of applications, including finished articles such as tires.

The 3-pyridazine compounds of the present invention when utilized as primary accelerators with rubber have been found to yield very much improved cure rates and cure states, i.e., lower t25-t2 or t90-t2 values and higher Vmax values and higher Rmax values. The improved cure rate values were generally superior to the values obtained utilizing conventional thiazole sulfenamide primary accelerators such as N-cyclohexyl-2-benzothiazole sulfenamide, N-t-butyl-2-benzothiazole sulfenamide, N-t-butyl-2-benzothiazole sulfenimide and the like. However, it is also found to be advantageous to use the accelerators of the invention as auxiliary accelerators, in combination with other well-known conventional accelerators, which include thiurams, dithiocarbamates, guanidines, such as diphenylguanidine (DPG) or di-ortho-tolylguanidine (DOTG), the various thiazoles, such as 2-mercaptobenzothiazole and 2,2'-benzothiazole disulfide; benzothiazole sulfenamides and sulfenimides, such as N-cyclohexyl-2-benzothiazole sulfenamide, N,N-dicyclohexyl-2-benzothiazole sulfenamide, N-t-butyl-2-benzothiazole sulfenimide, N,N-diisopropyl-2-benzothiazole sulfenamide, N-oxydiethylene-2-benzothiazole sulfenamide, N-isopropyl-2-benzothiazole sulfenamide and N-t-butyl-2-benzothiazole sulfenamide. Other known accelerators can be used, such as phosphorus-sulfur based accelerators (e.g., zinc phosphorodithioate) and thiuram accelerators. Examples of conventional thiuram accelerators include N,N'-dimethyl-N,N'-diphenylthiuram disulfide, dipentamethylenethiuram hexasulfide, tetramethylthiuram monosulfide, tetrabenzylthiuram disulfide, tetrabutylthiuram disulfide, tetramethylthiuram disulfide, and metal salts of the corresponding dithiocarbamic acids, such as those of zinc, copper, tellurium, etc. Other accelerating additives which can be used include zinc salts, such as zinc (diisocyanate) diamine complexes. Especially good results are obtained when the accelerators of the invention are used in combination with 2-mercaptobenzothiazole (MBT).

From 0.1 to 0.5 phr of the accelerators of the invention can be used, together with larger amounts (from 0.2 to 2.0 phr) of one or more conventional accelerators. Conversely, a small (0.1 to 0.5 phr) amount of one or more conventional accelerators can be used with a larger amount of one of the accelerators of the invention.

The invention will be better understood by reference to the following examples in which all parts are by weight and all temperatures are in degrees Celsius, unless otherwise specified.

EXAMPLES

Various 3-pyridazine disulfides and sulfenamides of the present invention were tested in accordance with appropriate ASTM procedures for rubber. Parameters which characterize vulcanization were taken from ODR (oscillating disc rheometer) cure curves ("rheographs"), which were obtained for vulcanization at 150°, 153°, 160° or 175°. As is graphically shown in the drawing, the parameters Rmin and Rmax are the minimum rheometer torque (before the onset of vulcanization) and the maximum rheometer torque (due to vulcanization), respectively. The parameter t2 is the time required for an increase (over Rmin) in rheometer torque of 2.2dNm (2.0 in-lb); t25 is the time required for the occurrence of 25 percent of the increase in torque due to vulcanization (time at which torque equals (Rmax-Rmin)0.25 + Rmin); t90 is the time required for the occurrence of 90 percent of the increase in torque due to vulcanization (time at which torque equals (Rmax-Rmin)0.9 + Rmin). Vmax is the maximum slope of the vulcanization curve divided by Rmax-Rmin, expressed in terms of percent per minute.

The invention will be better understood by reference to the following examples in which all parts are per 100 parts by weight of rubber (phr) and all temperatures are in degrees Celsius, unless otherwise specified.

Preparation of Rubber Masterbatches for Accelerator Evaluation

The various examples of 3-pyridazine accelerators which were prepared were tested in typical NR and SBR carbon-black reinforced compounds.

An SBR rubber masterbatch was prepared, based on SBR-1500, containing the following ingredients:

| SBR Masterbatch | Parts |
| --- | --- |
| SBR-1500 | 100.0 |
| Carbon Black N-330 | 50.0 |
| Circosol 4240, a Naphthenic Oil, ASTM D2226, Type 103 | 10.0 |
| Zinc Oxide | 4.0 |
| Stearic Acid | 2.0 |
| | 166.0 |

The SBR masterbatch was prepared by mixing the above-noted components in a Banbury mixer according to standard techniques. Subsequently, various accelerators, sulfur, and an antidegradant were added on a laboratory roll mill in the amounts set forth hereinbelow and blended by using standard laboratory mill mixing techniques:

|  | Parts |
|---|---|
| SBR-Masterbatch | 166.0 |
| SANTOFLEX 13 | 2.0 |
| Sulfur | 2.0 |
| Accelerators | As indicated |

SBR-1500 is a cold emulsion-polymerized, non-pigmented styrene/butadiene copolymer rubber containing nominally 23.5 percent bound styrene;

SANTOFLEX® 13 is N-(1,3-dimethylbutyl)-N'-phenyl-para-phenylenediamine, an antidegradant.

In a similar manner, a natural rubber masterbatch was made:

| Natural Rubber Masterbatch | Parts |
|---|---|
| Natural Rubber (SMR-CV) | 100.0 |
| Carbon Black N-330 | 50.0 |
| Naphthenic Oil; Circosol 4240 | 5.0 |
| Zinc Oxide | 5.0 |
| Stearic Acid | 2.0 |
| Total | 162.0 |

The natural rubber masterbatch was blended with the following compounds according to standard laboratory mill-mixing techniques:

|  | Parts |
|---|---|
| Natural Rubber Masterbatch | 162.0 |
| SANTOFLEX 13 | 1.0 |
| Flectol H | 1.0 |
| Sulfur | 2.4 |
| Accelerators | As indicated |

The following Examples 1-11 show the preparation of a number of 3-pyridazyl compounds of the invention. Following this are Tables I-XI which set forth the test data for rubber compositions of the invention (and the control compositions).

EXAMPLE 1

N-t-Butyl-3-pyridazine Sulfenamide

To a reactor equipped with a mechanical stirrer, a condenser, a thermometer and a liquid inlet was charged 3-mercaptopyridazine (0.01 mole, 1.12 g) and t-butylamine (0.48 mole, 50 ml). The resulted mixture was stirred and fed sodium hypochloride (0.012 mole, 0.89 g from 16% aqueous solution) at 28-35°C over 30 minutes period. The reaction mixture was evaporated to a minimum volume, added 50 ml of water, filtered, washed with 25 ml of water and dried to give 1.65 g (90%) of the product: m. p. 139-140°C. NMR: ($\delta$, multiplicity, assignment, integration) 1.2, s, t-$C_4H_9$, 9 H; 3.0, broad, NH, 1 H; 7.3 - 8.8, m, heterocycle, 3H.

## EXAMPLE 2

N-t-Butyl-6-chloro-3-pyridazine Sulfenamide

The procedure of Example 1 was used. A stirred solution of 3-mercapto-6-chloropyridazine (0.031 mole, 4.5 g) in t-butylamine (0.96 mole, 100 ml) was fed sodium hypochlorite (0.04 mole, 3.04 g from 16% aqueous solution) at 30-35°C over one hour period. The mixture was evaporated to remove bulk of t-butylamine. The remained mixture was added 400 ml of ice water, stirred, filtered, washed with 200 ml of water and dried to give 6.1 g (91%) of the product: m. p. 122-123°C. NMR: ($\delta$, multiplicity, assignment, integration) 1.3, s, t-$c_4H_9$, 9 H; 3.1, broad, NH, 1 H; 7.3-7.8, m, heterocycle, 2 H.

## EXAMPLE 3

N-t-Butyl-6-methoxy-3-pyridazine Sulfenamide

The procedure of Example 1 was adopted except other modification noted. A solution of 3-mercapto-6-methoxypyridazine (0.01 mole, 1.42 g) in t-butylamine (0.48 mole, 50 ml) was stirred and fed sodium hypochlorite (0.012 mole, 0.89 g from 16% aqueous solution) at 30-35°C over 30 minutes period. The mixture was evaporated, added ice water (100 ml), filtered, washed with 50 ml of water and dried to give 1.73 g (81%) of the product: m. p. 91-92°C. NMR: ($\delta$, multiplicity, assignment, integration) 1.2, s, t-$C_4H_9$, 9 H, 3.1, broad, NH, 1 H; 4.1, s, $CH_3O$, 3 H and 6.8-7.7, m, heterocycle, 2 H.

## EXAMPLE 4

N-t-Butyl-6-Hydroxy-3-Pyridazine Sulfenamide

A mixture of 10.0 g of 6-hydroxy-3-mercaptopyridazine, 20.9 g of t-butylamine, 175 ml of methanol and 1.5 g of carbon catalyst was placed into a 300-ml autoclave and heated to 55°C under 0.24 MPa of oxygen pressure for 4.3 hours. The autoclave was cooled and vented, and the solution was filtered. The methanol filtrate was concentrated under reduced pressure and then cooled to 0°C. The resulting 8.7 g of N-t-butyl-6-hydroxy-3-pyridazine sulfenamide had a melting point of 139-144°C.

## EXAMPLE 5

3,3'-Di(6-Chloropyridazine) Disulfide

A mixture of 15.0 g of 6-chloro-3-mercaptopyridazine, 200 ml of isopropanol, and 2.0 g of carbon catalyst was placed into a 300 ml autoclave and heated to 35°C under 0.17 MPa of oxygen pressure for one hour. The autoclave was cooled and vented, and the solution was filtered. The carbon filter cake was then extracted with hot acetonitrile. On cooling, white platelets crystallized out and were collected by filtration. The yield of 3,3'-di(6-chloropyridazine) disulfide was 13.8 g, m.p. 137-140°C (with decomposition).

## EXAMPLE 6

3,3'-Di(6-Hydroxypyridazine) Disulfide

A mixture of 10.0 g of 6-hydroxy-3-mercaptopyridazine, 200 ml of isopropanol, and 2.0 g of carbon catalyst was placed into a 300-ml autoclave and heated to 55°C under 0.17 MPa of oxygen pressure for 30 minutes. The autoclave was cooled and vented, and the solution was filtered. The carbon filter cake was then extracted with hot methanol. On cooling, 8.9 g of 3,3'-di(6-hydroxypyridazine) disulfide crystallized out with a melting point of 208-210°C.

EXAMPLE 7

3,3'-Dipyridazine Disulfide

To a stirred mixture of 3-mercaptopyridazine (0.032 mol, 3.63 g) and aqueous sodium hydroxide (0.032 mol, 1.28 g) was added hydrogen peroxide (0.016 mol, 0.55 g in 30% aqueous solution) and dilute sulfuric acid (0.016 mol, 1.59 g) simultaneously at 40°C over one hour period. The resulted mixture was cooled to room temperature, filtered, washed with a small amount of water and dried to give 2.8 g (78%) of the product: m.p. 137-138°C. NMR spectrum and mass spectrum are consistent with the assigned structure.

EXAMPLE 8

N-Isopropyl-3-pyridazine Sulfenamide

A solution of 3-mercaptopyridazine (0.057 mol, 6.4 g) in isopropylamine (1.7 mol, 145 ml) was stirred and fed sodium hypochlorite (0.063 mol, 4.7 g from 15% aqueous solution) at 30-35°C over one hour period. The reaction mixture was evaporated to remove bulk of isopropylamine. The remaining mixture was cooled to 0°C and a solid precipitate formed. It was filtered, washed with a small amount of water and dried to give 7.7 g (80%) of the product: m.p. 45-46°. NMR: ($\delta$, multiplicity, assignment, integration) 1.2, d, $CH_3$, 6H; 3.20, m, CH, 1H; 3.48, broad, NH, 1H; 7.1-8.8, m, heterocycle, 3H.

EXAMPLE 9

N-Cyclohexyl-3-Pyridazine Sulfenamide

To a mixture of 3-mercaptopyridazine (0.1 mol, 11.2 g) and cyclohexylamine (0.3 mol, 29.5 g) was added sodium hypochlorite (0.12 mol, 8.9 g from 15% aqueous solution) at 30°C under stirring over 30 minutes period. The mixture was added to 200 g of cold water, filtered, washed with 10 ml of cold water and dried to give 10 g (48%) of the product: mp 82-84°C. NMR ($CDC1_3$): 1.22-2.02 m, $(CH_2)_5$, 10 H; 2.86, m, CH, 1H; 3.20, broad, NH, 1 H; 7.23-8.88, m, heterocycle, 3H.

EXAMPLE 10

6-Chloro-3-Mercaptopyridazine

To a stirred solution of 3,6-dichloropyridazine (0.67 mol, 100 g) in 500 ml of ethanol was added thiourea (0.067 mol, 5.1 g) at 5°C. Stirring was continued for 16 hours at room temperature. It was cooled to 5°C and an additional amount (0.067 mol, 5.1 g) of thiourea was added. It was stirred at room temperature for four hours. The resultant mixture was filtered and dried to give 7.63 g (25% based on thiourea) of isothiouronium chloride salt. The salt (0.034 mol, 7.63 g) was suspended in 100 ml of water and basified with saturated aqueous sodium carbonate to pH 11, stirred for 20 minutes, cooled to 10°C and basified with 25% aqueous sodium hydroxide to pH 12. A clear solution resulted. This was acidified with 6<u>N</u> hydrochloric acid to pH 3. The mixture was filtered, washed with a small amount of water, dried to give 4.5 g (90% yield based on the salt intermediate), m.p. 138-140°C. (Literature, 130-140°C)

EXAMPLE 11

6-Hydroxy-3-Mercaptopyridazine

A mixture of 75.0 g (0.58 mol) of 6-chloro-3-mercaptopyridazine, 52.3 g (0.67 mol) of sodium sulfide, 2.9 g of sulfur, 3.6 g of sodium hydroxide and 432.7 g of water was heated at reflux (100°C) for 88 hours. The solution was cooled to 25°C and acidified with 87.8 g of 37% HCl (aq). The yellow slurry was filtered to give 79.1 g of crude 6-hydroxy-3-mercaptopyridazine. The crude solid was slurried in acetone (750 ml) and filtered to remove salts. The acetone solution was evaporated to yield 60.0 g of the product which was identified by $^1$H and $^{13}$C NMR.

TABLE I

| COMPOUND OF EXAMPLE 1 IN SBR | | |
|---|---|---|
| Run # | 1 | 2 |
| SBR Masterbatch | 166 | 166 |
| TBBS | 1.2 | - |
| Compound Ex. 1 | - | 1.2 |
| Mooney Scorch, 135°, t5, min. | 27.3 | 12.3 |
| ODR Data @ 153° | | |
| Rmax, Nm | 4.32 | 4.80 |
| Rmin, Nm | 0.57 | 0.55 |
| t90, min. | 24.6 | 10.9 |
| t2, min. | 10.0 | 5.2 |
| t90-t2, min. | 14.6 | 5.8 |
| t25, min. | 13.8 | 6.4 |
| t25-t2 | 3.8 | 1.3 |
| Max. Veloc. of vulc., %/min. | 11.2 | 40.7 |

TABLE II

| COMPOUND OF EXAMPLE 1 IN NATURAL RUBBER | | |
|---|---|---|
| Run # | 3 | 4 |
| NR Masterbatch | 162 | 162 |
| TBBS | 0.6 | - |
| Compound Ex. 1 | - | 0.6 |
| Mooney Scorch, 120°, t5, min. | 31.6 | 16.5 |
| ODR Data @ 153° | | |
| Rmax, Nm | 3.86 | 4.43 |
| Rmin, Nm | 0.39 | 0.36 |
| t90, min. | 10.9 | 8.1 |
| t2, min. | 4.2 | 2.8 |
| t90-t2, min. | 6.7 | 5.3 |
| t25, min. | 5.5 | 3.3 |
| t25-t2, min. | 1.3 | 0.6 |
| Max. Veloc. of vulc, %/min. | 22.0 | 53.6 |
| Rever., % 30 min. | 20.2 | 20.7 |

TABLE III

| Compounds of Examples 2 and 3 in Natural Rubber and SBR | | | | | | |
|---|---|---|---|---|---|---|
| Run # | 5 | 6 | 7 | 8 | 9 | 10 |
| SBR Masterbatch | 166 | 166 | 166 | - | - | - |
| NR Masterbatch | - | - | - | 162 | 162 | 162 |
| TBBS | 1.2 | - | - | 0.6 | - | - |
| Compound Ex. 2 | - | 1.2 | - | - | 0.6 | - |
| Compound Ex. 3 | - | - | 1.2 | - | - | 0.6 |
| Mooney Scorch, t5, min. (SBR @ 135°, NR @ 121°C) | 65.4 | 39.4 | 49.8 | 31.7 | 21.5 | 23.6 |
| ODR Data @ 153°C | | | | | | |
| Rmax, Nm | 4.29 | 4.81 | 4.45 | 4.03 | 4.76 | 4.64 |
| Rmin, Nm | 0.60 | 0.57 | 0.58 | 0.52 | 0.48 | 0.49 |
| t90, min. | 26.2 | 18.5 | 28.4 | 12.8 | 10.4 | 12.1 |
| t2, min. | 11.2 | 7.0 | 8.6 | 5.1 | 3.8 | 4.1 |
| t90-t2, min. | 15.0 | 11.5 | 19.8 | 7.7 | 6.6 | 8.0 |
| t25, min. | 14.3 | 8.7 | 10.7 | 6.5 | 4.6 | 4.9 |
| t25-t2, min. | 3.1 | 1.7 | 2.1 | 1.4 | 0.8 | 0.8 |
| Max. Veloc of vulc., %/min. | 13.2 | 20.5 | 14.9 | 20.6 | 36.5 | 28.9 |
| Rever., %/30 min. | - | - | - | 26.6 | 28.6 | 29.1 |

The test results set forth in Tables I, II and III show varying effectiveness with the different compounds of the invention. In all instances, the compounds of the invention gave better (faster) cure rates and higher extents of cure than the control.

In order to evaluate the performance of the compounds of the invention, the compound of Example 1 was evaluated in standard EPDM rubber and acrylonitrile-butadiene rubber (NBR). Masterbatches of these two rubbers were prepared according to the following recipes:

| EPDM Masterbatch | Parts |
|---|---|
| Vistalon 5600 EPDM Rubber | 100.0 |
| Carbon Black N550 | 120.0 |
| Processing Oil Sunpar 2280 | 60.0 |
| Zinc Oxide | 3.0 |
| Stearic Acid | 2.0 |
| Total | 285.0 |

The techniques used above for the other masterbatches were followed, with the following additional materials added on the mill, as indicated in Table IV.

|  | Parts |
|---|---|
| EPDM Masterbatch | 285.0 |
| Sulfur | 2.0 |
| MBT Accelerator | 1.0 |
| Other accelerator | As indicated |

In a similar manner, a nitrile rubber (NBR) masterbatch was prepared as follows:

| NBR Masterbatch | Parts |
|---|---|
| Medium-acrylonitrile NBR, 70 Mooney | 100.0 |
| Carbon Black N326 | 30.0 |
| SRF Carbon Black N762 | 65.0 |
| Zinc Oxide | 5.0 |
| Stearic Acid | 1.0 |
| Plasticizer | 6.0 |
| Dispersing Aid[1] | 3.0 |
| Flectol® H Antidegradant[2] | 1.0 |
| Flectol® ODP Antidegradant[3] | 1.5 |
| Sulfur | 0.3 |
| Total | 212.8 |

1. Polar compounds and silica
2. Polymerized 1,2-dihydro-2,2,4-trimethylquinoline
3. Octylated Diphenylamine

To the above masterbatch, accelerators were added on the mill, as indicated in Table IV resulting in the indicated performance.

TABLE IV

| COMPOUND OF EXAMPLE 1 IN EPDM AND NBR | | | | | | |
|---|---|---|---|---|---|---|
| Run # | 11 | 12 | 13 | 14 | 15 | 16 |
| EPDM Masterbatch | 285.0 | 285.0 | - | - | - | - |
| NBR Masterbatch | - | - | 212.8 | 212.8 | 212.8 | 212.8 |
| Sulfur | 2.0 | 2.0 | - | 0.7 | 0.7 | 0.7 |
| Tetramethylthiuram Disulfide | 1.0 | - | 2.5 | - | - | - |
| 2-Mercaptobenzothiazole | 1.5 | 1.5 | - | 1.0 | 2.5 | - |
| Compound Example 1 | - | 1.0 | - | 1.5 | - | 2.5 |
| Mooney Scorch @ 121°C, t5,min. | - | - | 7.04 | 4.20 | 3.51 | 30.2 |
| ODR Data @ 175°(EPDM) 150°(NBR) | | | | | | |
| Rmax, Nm | 5.32 | 5.38 | 6.10 | 7.22 | 4.75 | 7.06 |
| Rmin, Nm | 0.68 | 0.71 | 0.93 | 1.05 | 0.99 | 0.98 |
| t90, min. | 5.59 | 9.97 | 8.34 | 8.84 | 13.81 | 21.34 |
| t2, min. | 1.07 | 1.43 | 1.99 | 1.28 | 1.34 | 5.98 |
| t90-t2, min. | 4.52 | 8.54 | 6.35 | 7.56 | 12.47 | 15.36 |
| t25, min. | 1.47 | 2.02 | 3.37 | 1.71 | 1.94 | 8.50 |
| t25-t2, min. | 0.40 | 0.59 | 1.38 | 0.43 | 0.60 | 2.52 |
| Max. Veloc. of vulc., %/min. | 80.6 | 50.6 | 18.2 | 91.9 | 36.8 | 17.9 |

To illustrate the use of the compounds of the invention as auxiliary accelerators ("kickers") in rubber, in combination with known accelerators, a series of runs was made. In the first runs, small amounts (0.1 to 0.3 phr) of the compound of Example 1 were added to natural and SBR formulations containing TBBS. The data are shown in Table V. Another comparison added small amounts of the compound of Example 1 to formulations containing N-t-butyl-2-benzothiazole sulfenimide (TBSI). The data are shown in Table VI.

TABLE V

| COMPOUND OF EXAMPLE 1 AS KICKER IN SBR AND NATURAL | | | | | | | | |
|---|---|---|---|---|---|---|---|---|
| Run # | 17 | 18 | 19 | 20 | 21 | 22 | 23 | 24 |
| SBR Masterbatch | 166 | 166 | 166 | 166 | - | - | - | - |
| NR Masterbatch | - | - | - | - | 162 | 162 | 162 | 162 |
| TBBS | 1.2 | 1.2 | 1.2 | 1.2 | 0.6 | 0.6 | 0.6 | 0.6 |
| Compound Ex. 1 | - | 0.1 | 0.2 | 0.3 | - | 0.1 | 0.15 | 0.2 |
| Mooney Scorch, 135°C, T5 min. | 26.94 | 24.23 | 20.65 | 18.78 | 14.31 | 11.78 | 10.75 | 9.95 |
| ODR Data @ 153°C | | | | | | | | |
| Rmax, Nm | 4.02 | 4.13 | 4.27 | 4.38 | 3.55 | 3.91 | 4.04 | 4.23 |
| Rmin, Nm | 0.54 | 0.52 | 0.53 | 0.53 | 0.41 | 0.41 | 0.41 | 0.41 |
| t90, min. | 15.48 | 13.48 | 11.49 | 10.49 | 7.38 | 6.44 | 6.11 | 5.71 |
| t2, min. | 6.39 | 6.05 | 5.37 | 5.12 | 3.00 | 2.76 | 2.67 | 2.48 |
| t90-t2, min. | 9.09 | 7.43 | 6.12 | 5.37 | 4.38 | 3.68 | 3.44 | 3.23 |
| t25, min. | 8.93 | 8.22 | 7.15 | 6.77 | 3.81 | 3.45 | 3.30 | 3.09 |
| t25-t2, min. | 2.54 | 2.17 | 1.78 | 1.65 | 0.81 | 0.69 | 0.63 | 0.61 |
| Max. Veloc. of Vulc.,%/min. | 17.5 | 23.9 | 29.9 | 34.0 | 32.5 | 42.9 | 48.8 | 52.6 |
| Reversion, %/30 min. | - | - | - | - | 29.5 | 29.2 | 29.4 | 29.4 |

TABLE VI

| COMPOUND OF EXAMPLE 1 AS KICKER IN SBR AND NATURAL RUBBER | | | | |
|---|---|---|---|---|
| Run # | 25 | 26 | 27 | 28 |
| SBR Masterbatch | 166 | 166 | - | - |
| NR Masterbatch | - | - | 162 | 162 |
| TBSI | 1.2 | 1.2 | 0.6 | 0.6 |
| Compound Ex. 1 | - | 0.2 | - | 0.1 |
| Mooney Scorch, 135°C, t5, min. | 40.39 | 27.13 | 15.43 | 12.15 |
| ODR Data @ 153°C | | | | |
| Rmax, Nm | 4.17 | 4.51 | 3.12 | 3.49 |
| Rmin, Nm | 0.54 | 0.52 | 0.32 | 0.27 |
| t90, min. | 21.13 | 14.46 | 8.14 | 6.98 |
| t2, min. | 7.24 | 6.00 | 3.03 | 2.84 |
| t90-t2, min. | 13.89 | 8.46 | 5.11 | 4.14 |
| t25, min. | 12.05 | 8.84 | 4.32 | 3.80 |
| t25-t2, min. | 4.81 | 2.84 | 1.29 | 0.96 |
| Max. veloc. of Vulc., %/min. | 11.3 | 17.8 | 23.9 | 30.5 |
| Reversion, %/30 min. | - | - | 27.4 | 28.7 |

The N-isopropyl-3-pyridazine sulfenamide and the N-cyclohexyl-3-pyridazine sulfenamide (prepared in Examples 8 and 9) were similarly tested in SBR and natural rubber compounds. The results are shown in Tables VII, VIII, IX and X.

To show the effectiveness of 3,3'-dipyridazine disulfide and N-isopropyl-3-pyridazine sulfenamide in EPDM rubber, these compounds were compared with tetramethyl thiuram disulfide at the 1.0 phr level. Formulations and results are shown in Table XI.

TABLE VII

| COMPOUND OF EXAMPLES 8 IN SBR | | |
|---|---|---|
| Run # | 29 | 30 |
| SBR Masterbatch | 166 | 166 |
| TBBS | 1.2 | - |
| Compound Ex. 8 | - | 1.2 |
| Mooney Scorch, 135°C, t5, min. | 24.50 | 9.08 |
| ODR Data @ 153° | | |
| Rmax, Nm | 4.16 | 4.67 |
| Rmin, Nm | 0.56 | 0.55 |
| t90, min. | 14.84 | 6.45 |
| t2, min. | 5.87 | 2.92 |
| t90-t2, min. | 8.97 | 3.53 |
| t25, min. | 8.06 | 3.49 |
| t25-t2, min. | 2.19 | 0.57 |
| Max. Veloc. of vulc., %/min. | 18.1 | 73.8 |

TABLE VIII

| COMPOUND OF EXAMPLE 8 IN NATURAL RUBBER | | |
|---|---|---|
| Run # | 31 | 32 |
| NR Masterbatch | 162 | 162 |
| TBBS | 0.6 | - |
| Compound Ex. 8 | - | 0.6 |
| Mooney Scorch, 121°, t5, min. | 36.66 | 14.59 |
| ODR Data @ 153° | | |
| Rmax, Nm | 3.47 | 3.88 |
| Rmin. Nm | 0.35 | 0.30 |
| t90, min. | 7.15 | 4.90 |
| t2, min. | 3.05 | 1.97 |
| t90-t2, min. | 4.10 | 2.93 |
| t25, min. | 3.88 | 2.30 |
| t25-t2, min. | 0.83 | 0.33 |
| Max. Veloc. of vulc., %/min. | 33.9 | 73.5 |
| Reversion, %/30 min. | 30.5 | 35.2 |

TABLE IX

| COMPOUND OF EXAMPLE 9 IN SBR | | |
|---|---|---|
| Run # | 33 | 34 |
| SBR Masterbatch | 166.0 | 166.0 |
| TBBS | 1.2 | - |
| Compound Ex. 9 | - | 1.2 |
| Mooney Scorch, 135°C t5, min. | 27.45 | 13.87 |
| ODR Data @ 153° | | |
| Rmax, Nm | 4.35 | 4.62 |
| Rmin, Nm | 0.59 | 0.59 |
| t90, min. | 15.40 | 7.73 |
| t2, min. | 6.52 | 3.96 |
| t90-t2, min. | 8.88 | 3.77 |
| t25, min. | 9.28 | 4.79 |
| t25-t2, min. | 2.76 | 0.83 |
| Max. Veloc. of vulc., %/min. | 20.2 | 61.1 |

TABLE X

| COMPOUND OF EXAMPLE 9 IN NATURAL RUBBER | | |
|---|---|---|
| Run # | 35 | 36 |
| NR Masterbatch | 162.0 | 162.0 |
| TBBS | 0.6 | - |
| Compound Ex. 9 | - | 0.6 |
| Mooney Scorch, 121°C t5, min. | 42.93 | 21.11 |
| ODR Data @ 153° | | |
| Rmax, Nm | 3.65 | 3.96 |
| Rmin, Nm | .53 | .47 |
| t90, min. | 7.58 | 5.81 |
| t2, min. | 3.13 | 2.45 |
| t90-t2, min. | 4.45 | 3.36 |
| t25, min. | 4.23 | 2.88 |
| t25-t2, min. | 1.10 | 0.43 |
| Max. Veloc. of vulc., %/min. | 34.3 | 77.7 |

TABLE XI

| COMPOUNDS OF EXAMPLES 7 AND 8 IN EPDM | | | |
|---|---|---|---|
| Run # | 37 | 38 | 39 |
| EPDM Masterbatch | 285 | 285 | 285 |
| Sulfur | 2.0 | 2.0 | 2.0 |
| 2-Mercaptobenzothiazole | 1.5 | 1.5 | 1.5 |
| Tetramethyl Thiuram Disulfide | 1.0 | - | - |
| Compound, Ex. 7 | - | 1.0 | - |
| Compound, Ex. 8 | - | - | 1.0 |
| Mooney Scorch, 135°C, t5, min. | 4.81 | 10.35 | 6.13 |
| ODR Data @ 175°C | | | |
| Rmax, Nm | 5.49 | 5.19 | 5.21 |
| Rmin, Nm | 0.73 | 0.76 | 0.76 |
| t90, min. | 5.26 | 9.28 | 9.09 |
| t2, min. | 1.07 | 1.26 | 1.21 |
| t90-t2, min. | 4.19 | 8.02 | 7.88 |
| t25, min. | 1.50 | 1.78 | 1.71 |
| t25-t2, min. | 0.43 | 0.52 | 0.50 |
| Max. Veloc. of Vulc., %/min. | 82.7 | 51.0 | 51.9 |

## Claims

1. A composition comprising sulfur-vulcanizable rubber and from 0.1 to 10 parts by weight per 100 parts by weight of rubber of a compound of the formula

$$(PdS)_x(NRR')_yR''_z$$

wherein Pd is 3-pyridazyl, optionally substituted on the nucleus by one or more substituents selected from halogen atoms, lower alkoxy groups and hydroxyl groups; R is H, or $C_{1-8}$ alkyl, $C_{3-8}$ cycloalkyl, phenyl, $C_{7-12}$ aralkyl or $C_{7-12}$ alkaryl; R' is H or R, or R and R' together with N form a heterocyclic ring; X can be 2, y can be 0 and z can be 0.

2. The composition of Claim 1 also comprising from 0.1 to 5 parts by weight of sulfur, from 5 to 200 parts by weight of a filler and from 0.1 to 5 parts by weight of an antidegradant per 100 parts by weight of rubber.

3. The composition of Claim 2 also comprising from 5 to 200 parts by weight of oil.

4. The composition of Claim 1, wherein Pd is unsubstituted.

5. The composition of Claim 1, wherein Pd is 6-chloro-3-pyridazyl.

6. The composition of Claim 1, wherein Pd is 6-hydroxy-3-pyridazyl.

7. The composition of Claim 3, also including from 0.1 to 0.5 phr of a conventional accelerator.

16

8.  The composition of Claim 3 wherein the compound is present in an amount of from 0.1 to 0.5 parts by weight, and a conventional accelerator is also present, in an amount of from 0.2 to 2.0 parts by weight, per 100 parts by weight of rubber.

9.  3,3'-Dipyridazine disulfide.

10. 3,3'-Di(6-chloropyridazine)disulfide.

11. 3,3'-Di(6-hydroxypyridazine)disulfide.

VULCANIZATION PARAMETERS